# EUROPÄISCHE PATENTANMELDUNG

(11) **EP 3 023 488 A1**
(43) Veröffentlichungstag der Anmeldung: **25.05.2016**
(21) Anmeldenummer: 15195896.4
(22) Anmeldetag: 23.11.2015
(51) Int. Cl.: C12M 1/06, C12M 3/06, B01F 11/02

(54) **SCHÜTTELFERMENTERGEFÄSS SOWIE DESSEN VERWENDUNG**

(30) Priorität: 21.11.2014 DE 102014017189
(71) Anmelder: Infors AG, 4103 Bottmingen (CH)
(72) Erfinder: Hawrylenko, Alexander, 4103 Bottmingen (BL) (CH)
(74) Vertreter: Säger, Manfred

(57) **Zusammenfassung**

Um ein Schüttelfermentergefäß (1) mit einem Standboden (2) und mit einer diesem entgegen gesetzten Öffnung (3) so weiterzubilden, dass im Betrieb eine bessere Mischungswirkung erreicht werden kann, wird vorgeschlagen, dass bezogen auf den Standboden (2) im Inneren des Schüttelfermentergefäßes (1) eine Achse (6) aufgerichtet ist und dass mit radialem Abstand an der Achse (6) mindestens eine Schwungmasse (9) angeordnet ist.

## Beschreibung

### Technisches Gebiet

Die vorliegende Erfindung betrifft grundsätzlich das technische Gebiet der Schüttelfermentergefäße. Im Spezielleren betrifft die vorliegende Erfindung ein gattungsgemäßes Schüttelfermentergefäß gemäß dem Oberbegriff des Anspruchs 1, nämlich ein Schüttelfermentergefäß mit
- einem Standboden und
- einer zu diesem entgegengesetzt angeordneten Öffnung,
sowie eine entsprechende Verwendung.

### Stand der Technik

Derartige gattungsgemäße Schüttelfermentergefäße sind in einer Vielzahl von Ausführungsformen bekannt, haben sich bewährt und können noch weitere Merkmale aufweisen.

Sie weisen im Betrieb auf einem Schüttler allerdings für manche Anwendungszwecke eine nicht ausreichende Mischungswirkung auf, weshalb sie entweder länger betrieben oder aber mit einem zusätzlichen Rührer versehen werden müssen, was aufwändig ist. Hierbei ist auch ein gesonderter Antrieb für einen Rührer bekannt (vgl. Druckschrift US 3 384 354).

Zum technologischen Hintergrund der vorliegenden Erfindung kann auf die Druckschriften DE 855 238 B, DE 10 2008 058 338 A1, US 1 242 824, US 2 958 517, US 2008/0131957 A1 und WO 2011/091233 A1 aufmerksam gemacht werden.

### Darstellung der vorliegenden Erfindung: Aufgabe, Lösung, Vorteile

Ausgehend von den vorstehend dargelegten Nachteilen und Unzulänglichkeiten sowie unter Würdigung des umrissenen Stands der Technik liegt der vorliegenden Erfindung die Aufgabe zugrunde, ein Schüttelfermentergefäß gemäß dem Oberbegriff des Anspruchs 1 so weiterzubilden, dass damit im Betrieb eine bessere Mischungswirkung erreicht werden kann.

Diese Aufgabe wird bei einem Schüttelfermentergefäß gemäß dem Oberbegriff des Anspruchs 1 erfindungsgemäß durch dessen kennzeichnende Merkmale, also dadurch gelöst, dass bezogen auf den Standboden im Inneren des, insbesondere zylindrischen, Schüttelfermentergefäßes eine Achse, insbesondere im Wesentlichen rechtwinklig zum Standboden, aufgerichtet ist und dass mit radialem Abstand an der Achse mindestens eine Schwungmasse angeordnet ist.

Vorteilhafte Ausführungsformen der vorliegenden Erfindung sind in den abhängigen Ansprüchen gekennzeichnet; zweckmäßige Weiterbildungen der vorliegenden Erfindung sind in den nebengeordneten Ansprüchen gekennzeichnet.

Gemäß einer vorteilhaften Ausführungsform der vorliegenden Erfindung kann die Öffnung mit einem Deckel verschlossen sein, an dem vorzugsweise in einer Ausnehmung das eine Ende der Achse gelagert sein kann.

Zweckmäßigerweise kann die Ausnehmung
- im Inneren des Deckels und/oder
- mittig oder zentral im Deckel
angeordnet sein.

Das andere Ende der Achse kann in vorteilhafter Weise im Bereich des Standbodens in einer, insbesondere mittigen oder zentralen, Ausnehmung gelagert sein, die zweckmäßigerweise am Schüttelfermentergefäß selbst oder an einem dort eingesetzten gewölbten Blech vorgesehen sein kann.

Vorzugsweise ist die Schwungmasse selbst als paddelförmiger Rührer ausgebildet und/oder mit zumindest einem paddelförmigen, insbesondere rechteckigen, Rührer, zum Beispiel entweder über eine konzentrisch zur Achse angeordnete Hülse oder aber, insbesondere bei als sich drehende Welle ausgebildeter Achse, über diese selbst, starr verbunden.

Verfahrensgemäß wird das erfindungsgemäß ausgebildete Schüttelfermentergefäß auf eine eine kreisförmige Schüttelbewegung parallel zum Standboden ausführende Unterlage eines Schüttelgeräts gestellt.

Hierbei führt jeder Punkt dieser Unterlage und damit auch die radial zur Achse bzw. Welle versetzte (bezogen auf das ansonsten ruhende Schüttelgerät) Schwungmasse infolge der Zentrifugalkraft eine kreisende Bewegung um die Achse bzw. Welle aus, so dass jeder Punkt der Unterlage sich jeweils um einen Mittelpunkt bewegt und alle Punkte von ihrem jeweiligen Mittelpunkt denselben Durchmesser aufweisen.

Die Schwungmasse führt also infolge der Zentrifugalkraft eine Kreisbewegung um die Achse bzw. Welle aus und treibt den zumindest einen mit ihr bewegten paddelförmigen Rührer an, wodurch nicht nur die Schüttelbewegung, sondern auch der/die Rührer zu der infolgedessen besseren Durchmischung beiträgt/beitragen.

Die vorliegende Erfindung betrifft auch die Verwendung mindestens eines Schüttelfermentergefäßes gemäß der vorstehend dargelegten Art mit einem Schüttelgerät.

### Kurze Beschreibung der Zeichnungen

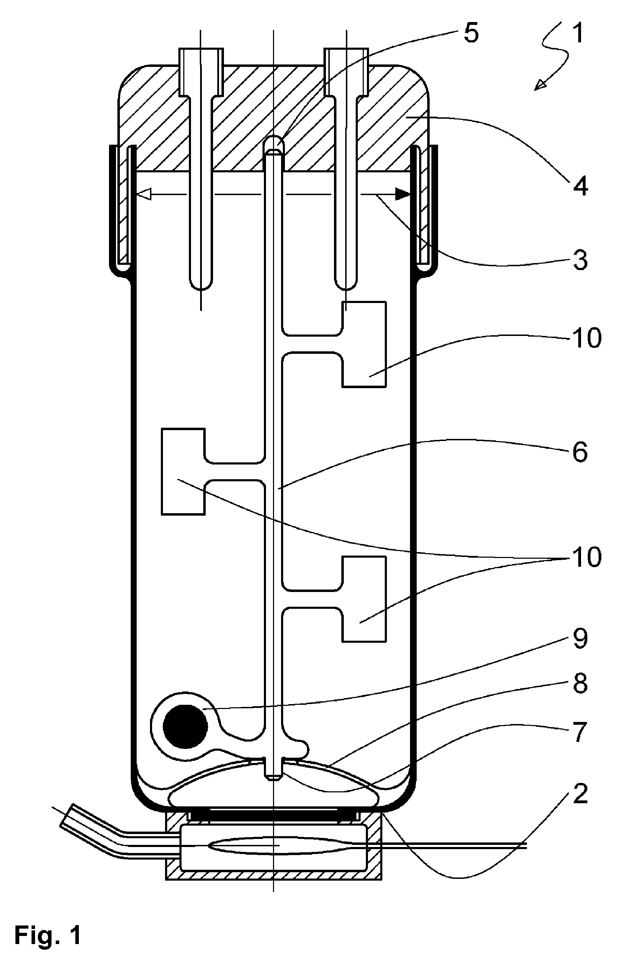

Wie bereits vorstehend erörtert, gibt es verschiedene Möglichkeiten, die Lehre der vorliegenden Erfindung in vorteilhafter Weise auszugestalten und weiterzubilden. Hierzu wird einerseits auf die dem Anspruch 1 sowie dem Anspruch 14 nachgeordneten Ansprüche verwiesen, andererseits werden weitere Ausgestaltungen, Merkmale und Vorteile der vorliegenden Erfindung nachstehend unter anderem anhand des durch Fig. 1 veranschaulichten Ausführungsbeispiels näher erläutert.

Es zeigt:
- Fig. 1: in schematischer Querschnittansicht ein Ausführungsbeispiel eines Schüttelfermentergefäßes gemäß der vorliegenden Erfindung.

Die Dimensionierung der einzelnen Elemente oder Merkmale, insbesondere in Relation zueinander, ist in Fig. 1 nicht notwendigerweise maßstabsgerecht.

### Bester Weg zur Ausführung der vorliegenden Erfindung

Ein insgesamt mit 1 bezeichnetes, zylinderförmig ausgebildetes Schüttelfermentergefäß weist einen Standboden 2 und eine dazu entgegen gesetzt angeordnete Öffnung 3 auf, die mittels eines Deckels 4 verschlossen werden kann.

Im Inneren des Deckels 4 ist mittig in einer Ausnehmung 5 das eine Ende einer im Inneren des Gefäßes 1 vorgesehenen Achse 6 gelagert, deren anderes Ende in einer mittigen Ausnehmung 7 eines in den Boden des Schüttelfermentergefäßes 1 eingesetzten gewölbten Blechs 8 rechtwinklig zum Standboden 2 gelagert ist.

Mit radialem Abstand zur als Welle ausgebildeten Achse 6 ist eine Schwungmasse 9 vorgesehen, die zudem als paddelförmiger Rührer ausgestaltet ist und die über die Welle 6 mit drei weiteren paddelförmigen Rührern 10 starr verbunden ist, zum Beispiel über eine konzentrisch zur Welle 6 angeordnete Hülse.

Wird das erfindungsgemäß ausgebildete Schüttelfermentergefäß 1 auf eine eine Schüttelbewegung ausführende Unterlage eines Schüttelgeräts gestellt, so führt jeder Punkt dieser Unterlage und damit auch die radial zur Achse 6 bzw. Welle versetzte Schwungmasse 9 (bezogen auf das ansonsten ruhende Schüttelgerät) eine Kreisbewegung - mit jeweils dem gleichen Radius - um einen Mittelpunkt aus.

Die Schwungmasse 9 führt infolge der Zentrifugalkraft eine Kreisbewegung um die Achse 6 bzw. Welle aus und treibt die mit ihr bewegten paddelförmigen Rührer 10 an, was zu einer besseren Durchmischung im Betrieb führt.

### Bezugszeichenliste

1 Schüttelfermentergefäß
2 Standboden
3 Öffnung
4 Deckel
5 Ausnehmung im Inneren des Deckels 4
6 Achse, insbesondere Welle
7 Ausnehmung im Bodenbereich des Schüttelfermentergefäßes 1
8 in den Bodenbereich des Schüttelfermentergefäßes 1 eingesetztes gewölbtes Blech
9 Schwungmasse
10 paddelförmiger, insbesondere rechteckiger, Rührer

## Patentansprüche

1. Schüttelfermentergefäß (1) mit
- einem Standboden (2) und
- einer diesem entgegen gesetzten Öffnung (3),
**dadurch gekennzeichnet,**
- **dass** bezogen auf den Standboden (2) im Inneren des Schüttelfermentergefäßes (1) eine Achse (6) aufgerichtet ist und
- **dass** mit radialem Abstand an der Achse (6) mindestens eine Schwungmasse (9) angeordnet ist.

2. Schüttelfermentergefäß gemäß Anspruch 1, **dadurch gekennzeichnet, dass** die Öffnung (3) mit einem Deckel (4) verschlossen ist, an dem in einer Ausnehmung (5) das eine Ende der Achse (6) gelagert ist.

3. Schüttelfermentergefäß gemäß Anspruch 2, **dadurch gekennzeichnet, dass** die Ausnehmung (5) im Inneren des Deckels (4) angeordnet ist.

4. Schüttelfermentergefäß gemäß Anspruch 2 oder 3, **dadurch gekennzeichnet, dass** die Ausnehmung (5) mittig oder zentral im Deckel (4) angeordnet ist.

5. Schüttelfermentergefäß gemäß mindestens einem der Ansprüche 1 bis 4, **dadurch gekennzeichnet, dass** das andere Ende der Achse (6) im Bereich des Standbodens (2) in einer Ausnehmung (7) gelagert ist, die am Schüttelfermentergefäß (1) selbst oder an einem dort eingesetzten gewölbten Blech (8) vorgesehen ist.

6. Schüttelfermentergefäß gemäß Anspruch 5, **dadurch gekennzeichnet, dass** die Ausnehmung (7) mittig oder zentral angeordnet ist.

7. Schüttelfermentergefäß gemäß mindestens einem der Ansprüche 1 bis 6, **dadurch gekennzeichnet, dass** die Achse (6) rechtwinklig zum Standboden (2) aufgerichtet ist.

8. Schüttelfermentergefäß gemäß mindestens einem der Ansprüche 1 bis 7, **dadurch gekennzeichnet, dass** die Achse (6) als Welle ausgebildet ist.

9. Schüttelfermentergefäß gemäß mindestens einem der Ansprüche 1 bis 8, **dadurch gekennzeichnet, dass** die Schwungmasse (9) mit mindestens einem paddelförmigen Rührer (10) starr verbunden ist.

10. Schüttelfermentergefäß gemäß mindestens einem der Ansprüche 1 bis 9, **dadurch gekennzeichnet, dass** die Schwungmasse (9) als paddelförmiger Rührer ausgebildet ist.

11. Schüttelfermentergefäß gemäß Anspruch 9 oder 10, **dadurch gekennzeichnet, dass** die Schwungmasse (9) mit dem paddelförmigen Rührer (10) über die Achse (6) verbunden ist.

12. Schüttelfermentergefäß gemäß mindestens einem der Ansprüche 9 bis 11, **dadurch gekennzeichnet, dass** die Schwungmasse (9) mit dem paddelförmigen Rührer (10) über eine konzentrisch zur Achse (6) angeordnete Hülse verbunden ist.

13. Schüttelfermentergefäß gemäß mindestens einem der Ansprüche 1 bis 12, **gekennzeichnet durch** eine zylinderförmige Ausbildung.

14. Verwendung mindestens eines Schüttelfermentergefäßes (1) gemäß mindestens einem der Ansprüche 1 bis 13 mit einem Schüttelgerät.

15. Verwendung gemäß Anspruch 14, **gekennzeichnet durch** eine eine kreisförmige Schüttelbewegung parallel zum Standboden (2) ausführende Unterlage des Schüttelgeräts.
